(19) Europäisches Patentamt
European Patent Office
Office européen des brevets

(11) **EP 4 187 544 A1**

(12) **EUROPEAN PATENT APPLICATION**

(43) Date of publication:
**31.05.2023 Bulletin 2023/22**

(21) Application number: **22209795.8**

(22) Date of filing: **28.11.2022**

(51) International Patent Classification (IPC):
**G16B 25/00** (2019.01)    **G16B 20/00** (2019.01)
**G16B 40/20** (2019.01)    **G16B 20/40** (2019.01)
**C12Q 1/68** (2018.01)

(52) Cooperative Patent Classification (CPC):
**G16B 40/20; G16B 20/00; G16B 20/40;
G16B 25/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **29.11.2021 US 202163283910 P**

(71) Applicant: **The Chinese University of Hong Kong
Shatin, New Territories (HK)**

(72) Inventors:
• **WANG, Maggie Haitian
Shatin (CN)**
• **ZHENG, Hong
Shatin (CN)**
• **ZEE, Benny Chung-Ying
Homantin (CN)**

(74) Representative: **Geling, Andrea
ZSP Patentanwälte PartG mbB
Hansastraße 32
80686 München (DE)**

(54) **CODON DE-OPTIMIZATION OR OPTIMIZATION USING GENETIC ARCHITECTURE**

(57)    Replacement codons for modifying a genetic sequence are selected based on genetic architecture of a genome. For example, a location-specific estimation of codon usage can be generated, and preferred or un-preferred codons for a particular location can be identified statistically. A codon at a particular location can be replaced by a more-preferred or less-preferred synonymous codon. These techniques can be extended to replacement of $k$-mers of arbitrary length $k$ within a segment of length s, where s is at least equal to $k$.

*200*

*FIG. 2*

**Description**

CROSS-REFERENCE TO RELATED APPLICATION

This application claims the benefit of U.S. Provisional Application No. 63/283,910, filed November 29, 2021, the disclosure of which is incorporated herein by reference.

BACKGROUND

This disclosure relates generally to modification of genetic sequences and in particular to replacement of codons (or other nucleotide groups) with other codons (or other nucleotide groups).

A codon is a sequence of three nucleotides that encodes a specific amino acid residue in a polypeptide chain. Given four nucleotides (A, C, G, and T for DNA; A, C, G, and U for RNA), 64 codons are available. Three of the codons are stop codons, which indicate a termination of translation. The other 61 each encode one of 20 amino acid residues. Two amino acid residues (methionine and tryptophan) have a single corresponding codon, while each of the other 18 has at least two and as many as six synonymous codons.

Synonymous codons occur with different frequencies in a genome, and significant differences in the relative frequencies of synonymous codons have been observed between organisms. It is generally understood that replacement of a codon with a synonymous codon can affect RNA processing, gene expression, and protein folding, among other effects. Accordingly, different synonymous codons may affect replicative fitness of an organism, and synonymous recoding strategies (selectively replacing one or more codons with a synonymous codon) have been developed. Synonymous recoding strategies include codon optimization and codon de-optimization. Codon de-optimized sequences can be used, for example, to reduce replicative fitness of an organism for improved antigen degeneration and safety, which has application to production of live-attenuated vaccines. Conversely, codon optimized sequences can be used to increase replicative fitness of an organism to achieve higher efficiency of replication. Codon optimized sequences are frequently used to enhance the yield of antigens in the production of vaccines in selected organisms (cell lines, eggs, virus expression systems, and so on).

Existing codon de-optimization strategies involve replacing a preferred (frequently occurring) codon with an un-preferred (rarely occurring) synonymous codon, where preferred and un-preferred codons are identified by analyzing frequency of codons across the genome of the pathogen or the host. A related strategy involves replacing pairs of adjacent codons rather than single codons. This approach can adjust CpG or UpA dinucleotide content, which is known to affect gene expression. Another related strategy involves directly increasing CpG and UpA content. Conversely, codon optimization generally involves replace un-preferred codons with preferred codons. As with codon de-optimization, preferred and un-preferred codons are identified by analyzing frequency of codons across the genome of the pathogen or the host.

SUMMARY

Existing techniques to identify preferred and un-preferred codons have been based on a genome-wide analysis of the codon usage bias of the organism, e.g., counting the number of instances of each synonymous codon in the organism's genome without consideration of codon location with the genome or within a particular gene. However, synonymous codons may exhibit distinct usage or roles at different positions within a genome or even a gene, and epistatic interactions may occur among codons within and between genes. Consequently, an approach to codon replacement that does not consider the location of a codon within a genome may result in undesired effects, e.g., increasing rather than decreasing reproductive fitness (or vice versa).

Certain embodiments of the present invention relate to techniques for selecting replacement codons based on genetic architecture of a genome. For example, a location-specific estimation of codon usage in a genetic sequence (e.g., a genome or a portion thereof) can be generated, and more-preferred or less-preferred codons for a particular location can be identified statistically. A codon at a particular location can be replaced by a more-preferred or less-preferred synonymous codon. This approach can more reliably result in a desired outcome such as increasing or decreasing the reproductive fitness of an organism such as a pathogen. In some embodiments, epistatic interactions can be considered, and codon pairs (which may be adjacent or non-adjacent codon pairs) that exhibit statistical correlations can be replaced as pairs. In various embodiments, techniques described herein can be extended to replacement of $k$-mers of arbitrary length $k$ within a segment of length $s$, where s is at least equal to $k$.

Certain embodiments relate to methods of modifying a genome. Such methods can include: obtaining a plurality of samples of a genetic sequence of a target organism; determining, for each of a plurality of target locations in the genetic sequence, a location-specific probability score for each of a plurality of synonymous codons; and for each target location: selecting, based on the location-specific probability scores for the target location, a replacement codon; and

replacing, in a genomic molecule, an existing codon at the target location with the replacement codon.

In these and other embodiments, determining the probability score for a particular synonymous codon includes determining a fraction of the samples of the genetic sequence that include the particular synonymous codon at the target segment.

In these and other embodiments, the replacement codon can be a codon having a highest probability score among the synonymous codons at the target segment. Alternatively, the replacement codon can be a codon having lowest probability score among the synonymous codons at the target segment.

In these and other embodiments, methods can also include: computing, for each of a plurality of pairs of locations in the genetic sequence, a linkage disequilibrium parameter; and selecting at least some of the target locations based on the linkage disequilibrium parameter. For example, the target locations can be selected such that each target location has a linkage disequilibrium with respect to at least one other target location that is above a threshold.

In these and other embodiments, the target locations can include every location for which two or more synonymous codons exist, or any subset of the set of locations for which two or more synonymous codons exist.

In these and other embodiments, the target organism can be a pathogen.

In these and other embodiments, the target organism can be a virus and the location-specific probability scores can be determined based on samples of the virus genetic sequence obtained from host organisms belonging to a first species. For instance, the method can also include determining a global probability score for each of a plurality of synonymous codons based on samples of the virus genetic sequence obtained from host organisms belonging to a second species, wherein the replacement codon is selected based in part on the location-specific probability scores and based in part on the global probability scores. The method can further include: computing, for each of a plurality of pairs of locations in the genetic sequence, a linkage disequilibrium parameter; and selecting at least some of the target locations based on the linkage disequilibrium parameter.

Certain embodiments relate to methods of modifying a genome. Such methods can include: obtaining a plurality of samples of a genetic sequence of a target organism; determining, for each of a plurality of target segments in the genetic sequence, a probability score for each of a set of synonymous segments, wherein a synonymous segment is a segment obtained by replacing a $k$-mer in the target segment with a different $k$-mer without affecting a corresponding amino acid sequence, wherein each target segment has a length $s$ and $s \geq k$; and for each target segment: selecting, based on the probability scores for the target segment, a replacement segment from the set of synonymous segments; and replacing, in a genomic molecule, the target segment with the replacement segment.

In these and other embodiments, determining the probability score for a synonymous segment can include determining a sum of available $k$-mers in the segment, weighted by the $k$-mer frequencies observed in the samples.

In these and other embodiments, the replacement segment can be a segment that has a highest probability score among the synonymous segments at the target segment. Alternatively, the replacement segment has a lowest probability score among the synonymous segments at the target segment.

In these and other embodiments, various values of $k$ can be chosen. In some embodiments, the value of $k$ can be equal to 3, and each $k$-mer can correspond to a codon. In some alternative embodiments, the value of $k$ can be equal to 2, and each $k$-mer can correspond to a dinucleotide. In some alternative embodiments, the value of $k$ can be equal to 6, and each $k$-mer can correspond to a pair of adjacent codons.

In these and other embodiments, the method can also include: computing, for each of a plurality of pairs of segments in the genetic sequence, a linkage disequilibrium parameter; and selecting at least some of the target segments based on the linkage disequilibrium parameter.

In these and other embodiments, the target segments can be selected such that each target segment has a linkage disequilibrium with respect to at least one other target segment that is above a threshold.

In these and other embodiments, the target segments include every segment for which two or more synonymous segments exist, or any subset of the set of segments for which two or more synonymous segments exist.

In these and other embodiments, the target organism can be a pathogen.

The following detailed description, together with the accompanying drawings, will provide a better understanding of the nature and advantages of the claimed invention.

BRIEF DESCRIPTION OF THE DRAWINGS

FIGs. 1A and 1B illustrate the concept of synonymous codons as used herein.

FIG. 2 shows a flow diagram of a process for modifying a genome according to some embodiments.

FIG. 3 shows a flow diagram of a process for modifying a genome according to some embodiments.

FIG. 4 shows an example of a contingency table for two positions in a genetic sequence.

FIG. 5 shows a flow diagram of a process for selecting target locations for codon replacement according to some embodiments.

FIG. 6 shows a table illustrating differences in codon replacement using different methods.

FIG. 7 is a table showing, for each of five different codon replacement methods, a maximum number (and percentage) of codons that can be replaced using that method.

FIG. 8 is a table illustrating Hamming distance between sequences generated from a target sequence according to different codon replacement methods at their maximum recoding settings.

DETAILED DESCRIPTION

The following description of exemplary embodiments of the invention is presented for the purpose of illustration and description. It is not intended to be exhaustive or to limit the claimed invention to the precise form described, and persons skilled in the art will appreciate that many modifications and variations are possible. The embodiments have been chosen and described in order to best explain the principles of the invention and its practical applications to thereby enable others skilled in the art to best make and use the invention in various embodiments and with various modifications as are suited to the particular use contemplated.

Certain embodiments of the present invention relate to techniques for selecting replacement codons based on genetic architecture of a genome. For example, a location-specific estimation of codon usage in a genetic sequence (e.g., a genome or a portion thereof) can be generated, and more-preferred or less-preferred codons for a particular location can be identified statistically. A codon at a particular location can be replaced by a more-preferred or less-preferred synonymous codon. This approach can more reliably result in a desired outcome such as increasing or decreasing the reproductive fitness of an organism such as a pathogen. In some embodiments, epistatic interactions can be considered, and codon pairs (which may be adjacent or non-adjacent codon pairs) that exhibit statistical correlations can be replaced as pairs. In various embodiments, techniques described herein can be applied to codons, codon pairs, or more generally to $k$-mers (where a $k$-mer is a sequence of k nucleotides).

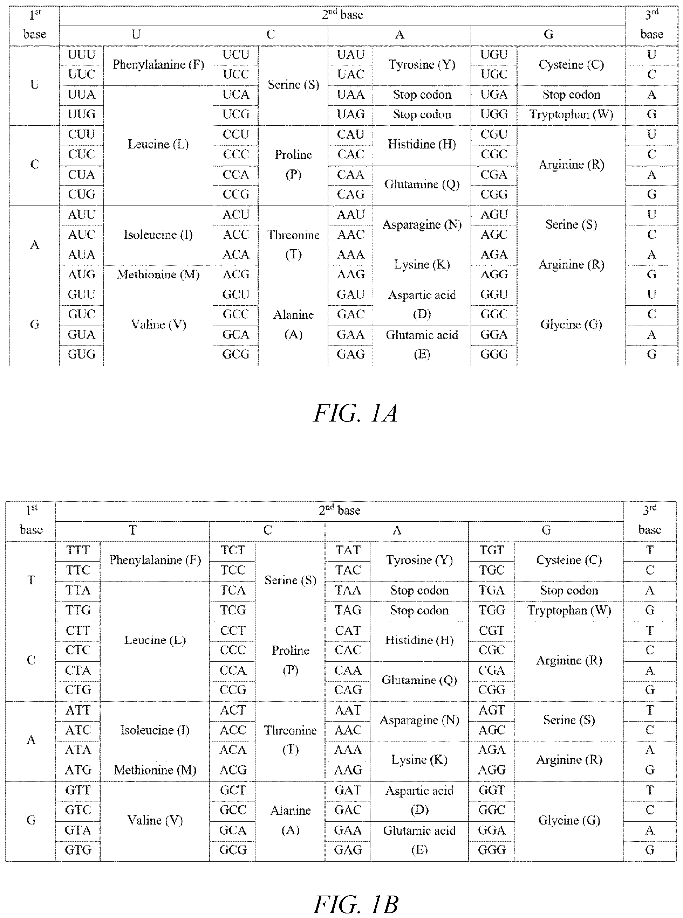

FIGs. 1A and 1B illustrate the concept of synonymous codons as used herein. FIG. 1A shows a codon table for RNA that maps each codon to the corresponding amino acid residue, and FIG. 1B shows the corresponding table for DNA. The nucleotide bases are represented using the usual convention: adenine (A), cytosine (C), guanine (G), thymine (T), and uracil (U). As shown, there are 64 codons, including three stop codons, one codon for tryptophan, and one codon for methionine. All other amino acids have multiple corresponding codons, referred to herein as "synonymous" codons.

While synonymous codons map to the same amino acid, different synonymous codons may have different effects in areas such as RNA processing, gene expression, and protein folding. Due to such effects, replacement of a particular codon in the genetic sequence of an organism with a synonymous codon may alter properties of the organism, including reproductive fitness.

**Position-Based Codon Replacement**

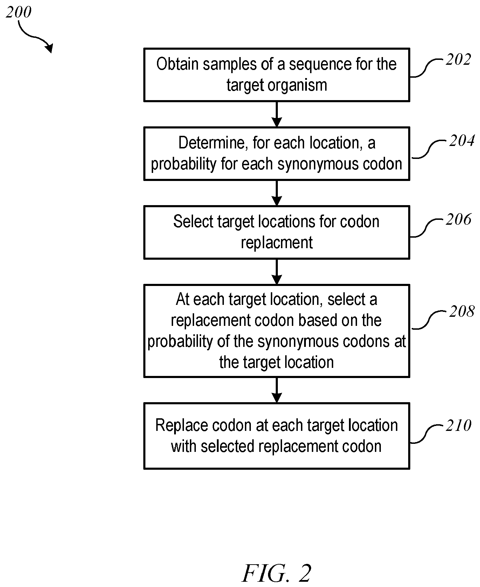

Certain embodiments disclosed herein provide techniques for selecting replacement codons (or more generally replacement $k$-mers) in a manner that increases the probability of achieving a desired effect on reproductive fitness without altering the encoded amino acid sequence. FIG. 2 shows a flow diagram of a process 200 for modifying a genome according to some embodiments. Process 200 can be performed for a variety of organisms, including pathogens such as viruses.

At block 202, samples of a genetic sequence for a target organism whose genome is to be modified are obtained. The target organism can be, for example, a virus or other pathogen. Genetic sequences for an organism can be obtained using conventional techniques for extracting and sequencing DNA or RNA, and the genetic sequence can include a portion or all of the genome of the target organism. Samples can be extracted from individual organisms and sequenced. For some organisms (e.g., various strains of influenza virus), genetic databases are available and can be used.

In notation used herein, it is assumed that a number (N) of samples are obtained. Samples are distinguished by a sample index $i$ (where $1 \leq i \leq N$). Each sample has a codon sequence { $X_j^i$ , $1 \leq j \leq J$}, where index $j$ represents a codon location (or codon position) within the sequence, $J$ is a total number of codons in the sequence and $X_j^i$ denotes the codon at the $j$th location in the ith sample. $A_j^0$ denotes the amino acid corresponding to the codon at the $j$th position in the target sequence.

At block 204, for each codon location $j$, a probability score (e.g., frequency of occurrence) can be determined for each synonymous codon. In some embodiments, a set of synonymous codons can be defined as { $X_j^0(r)$ , $1 \le r \le R_j$}, where index $r$ identifies a particular synonym (a codon that codes for amino acid $A_j^0$ ) and $R_j$ denotes the number of synonyms for codon $X_j^i$ . (The number $R_j$ of synonyms depends on the particular codon $X_j^i$ . For instance, as shown in FIG. 1B, ATG is the only codon for methionine, yielding $R_j = 1$. On the other hand, codons TTA, TTG CTT, CTC, CTA, and CTG all code for leucine, yielding $R_j = 6$.

In some embodiments, a probability score $p_j$ (r) can be computed from the $N$ samples according to:

$$ p_j(r) = \sum_{i=1}^{N} I\big(X_j^0(r) = X_j^i\big) /N , \qquad (1) $$

where $I(\cdot)$ is an identity function that is equal to 1 if the condition $(\cdot)$ is satisfied, 0 otherwise. In other words, the probability score in Eq. (1) can be the fraction of samples in which the codon $X_j^0(r)$ is present at location $j$. Other probability scores can also be defined. In this manner, a codon bias profile can be established for the organism, where the codon bias profile identifies more-preferred and less-preferred codons for each location.

At block 206, a set of target locations to be modified can be selected. The set of target locations can be represented as $\varphi$, and the number of target locations can be represented as $|\varphi|$. In some embodiments, every codon location can be selected as a target location, in which case $|\varphi| = J$. In other embodiments, the target locations can be a proper subset of the total number of codon locations, in which case $|\varphi| < J$. Selection of target locations can be random, or the selection can be based on prior biological information. For instance for some pathogens, information as to the effect of codon modifications at some locations may be available, and such information can be used to select target locations associated with a desired effect on the organism. Selection of target locations can also be based on statistical information. For instance, the range of probability scores for the synonymous codons at a given location may be considered, on the theory that where all synonymous codons with equal probability, replacement with a synonymous codon is likely to have negligible effect, but where the probabilities of different codons deviate from chance, a particular codon at that position may be beneficial (or detrimental) to the organism. As another example, codon locations where the amino acid has a unique codon ($R_j = 1$) and/or codon locations where a stop codon is present may be omitted from the set of target locations. Other considerations can also be applied.

At block 208, for each target location, a replacement codon is selected. In some embodiments, the replacement codon can be selected based on the probability scores and a desired effect of replacement. For example, a most-preferred codon $X_j^0(H)$ for a particular location $j$ can be defined as the codon for amino acid $A_j^0$ that most frequently occurs at location $j$. In some embodiments, the index $H$ for the most-preferred synonymous codon can be determined according to:

$$ H = \arg\max\big\{r|p_j(r), 1 \le r \le R_j\big\} . \qquad (2) $$

Consistent with Eq. (1), the probability score for the most-preferred synonymous codon can be defined as:

$$p_j(H) = \sum_{i=1}^{N} I\left(X_j^0(H) = X_j^i\right)/N \, . \tag{3}$$

For a given codon $X_j^0$ , the set ($\mathcal{H}$) of codons that are no less preferred than $X_j^0$ for amino acid $A_j^0$ can be defined as:

$$\mathcal{H} = \left\{X_j^0(r)|p_j(r) \geq p_j^0\right\}, \tag{4}$$

where $p_j^0$ is determined according to Eq. (1) with $X_j^0(r) = X_j^0$ . It should be understood that $X_j^0(H) \in \mathcal{H}$ .

Similarly, a least-preferred codon $X_j^0(L)$ for a particular location $j$ can be defined as the codon for amino acid $A_j^0$ that least frequently occurs at location $j$. In some embodiments, the index $L$ for the least-preferred synonymous codon can be determined according to:

$$L = \arg\min\left\{r|p_j(r), 1 \leq r \leq R_j\right\}. \tag{5}$$

Consistent with Eq. (1), the probability score for the least-preferred synonymous codon can be defined as:

$$p_j(L) = \sum_{i=1}^{N} I\left(X_j^0(L) = X_j^i\right)/N \, . \tag{6}$$

For a given codon $X_j^0$ , the set ($\mathcal{L}$) of codons that are no more preferred than $X_j^0$ for amino acid $A_j^0$ can be defined as:

$$\mathcal{L} = \left\{X_j^0(r)|p_j(r) \leq p_j^0\right\}, \tag{7}$$

where $p_j^0$ is determined according to Eq. (1) with $X_j^0(r) = X_j^0$ . It should be understood that $X_j^0(L) \in \mathcal{L}$ .

It should be noted that indexes $H$ and $L$ are position-specific, as are the sets $\mathcal{H}$ and $\mathcal{L}$ . In general, different synonymous codons for the same amino acid may be most preferred (or least preferred) at different positions in the sequence.

In some embodiments, codon optimization can be performed by selecting the most-preferred codon (e.g., the codon with $r = H$) for each target location. For example, on the assumption that the most-preferred codon correlates with reproductive fitness, the most-preferred codon for the target location can be selected in instances where enhancement of reproductive fitness is desired. In other embodiments, codon de-optimization can be performed by selecting the least-preferred codon (e.g., the codon with $r = L$) for each target location. For example, on the assumption that the least-preferred codon correlates with lack of reproductive fitness, the least-preferred codon can be selected in instances where reduction of reproductive fitness is desired. In still other embodiments, different selections can be made. As with the selection of target locations, prior biological information can be used in selecting replacement codons.

It should be noted that selection of a replacement codon is made for each location. In some embodiments, replacement codons for each location can be selected independently, e.g., based on the probability of different codons at that location. Thus, for example, if the target locations include two different locations that code for threonine, it is possible that ACG is selected as the replacement codon for the first location while ACC is selected as the replacement codon for the second location.

At block 210, for at least one instance of the organism, replacement of codons can be performed. In particular, at each target location, the existing codon can be replaced by the replacement codon selected for that location at block 208. Replacement of codons can be performed using existing techniques, such as designing appropriate primers for PCR (polymerase chain reaction) or other amplification reactions. In addition or instead, any specific polynucleotide sequence (such as a modified sequence determined at block 208) can be chemically synthesized, especially if it is of a relatively shorter length.

In various embodiments, process 200 can be applied to perform position-based codon de-optimization or codon optimization. In either case, the selection of replacement codon can be based on a position-specific probability score (e.g., according to Eq. (1)). The assumption that a higher position-specific probability score correlates with increased reproductive fitness, while a lower position-specific probability score correlates with decreased reproductive fitness, can be used to select replacement codons at specific positions.

For example, for codon de-optimization, a proportion of planned replacement ($0 < \pi \le 1$) can be selected, and a subset of codon positions can be chosen as the target locations $\psi$ such that $\pi = |\psi|/J$, where $|\psi|$ is the number of target locations. For example, if $\pi = 0.8$, then 80% of the residues in the target sequence would be selected. At each target location $j \in \psi$, the replacement $X_j^0 \leftarrow X_j^0(l)$ is performed, where $X_j^0(l) \in \mathcal{L}$. In other words, at each target location $j \in \psi$, the original codon is replaced with a codon that is the same or less preferred. In some embodiments, $l = L$ can be selected, which results in the replacement $X_j^0 \leftarrow X_j^0(L)$ at each target location. An actual proportion of de-optimization ($\varphi_{cd}$) can be used to represent the proportion of synonymous replacement conducted for $j \in \psi$ using the replacement $X_j^0 \leftarrow X_j^0(l)$. In some instances, the amino acid at a selected target location may correspond to a unique codon, in which case no replacement occurs. (This may be the case, e.g., if target locations are selected randomly.) Similarly, in some instances, the original codon at a particular position may already be the target codon (i.e., $X_j^0 = X_j^0(l)$), in which case no replacement occurs. Accordingly, it should be understood that, in a given application, $\varphi_{cd} \le \pi$.

Likewise, for codon optimization, a proportion of planned replacement ($0 < \pi \le 1$) can be selected, and a subset of codon positions can be chosen as the target locations $\psi$ such that $\pi = |\psi|/J$. At each location $j \in \psi$, the replacement $X_j^0 \leftarrow X_j^0(h)$ is performed, where , where $X_j^0(h) \in \mathcal{H}$. In other words, at each target location $j \in \psi$, the original codon is replaced with a codon that is the same or more preferred. In some embodiments, $h = H$ can be selected, which results in the replacement $X_j^0 \leftarrow X_j^0(H)$ at each target location. A proportion of optimization ($\varphi_{co}$) can be used to represent the proportion of synonymous replacement conducted for $j \in \psi$ using the replacement $X_j^0 \leftarrow X_j^0(h)$. As with codon de-optimization, in some instances, the amino acid at a selected target location may correspond to a unique codon, in which case no replacement occurs. (This may be the case, e.g., if target locations are selected randomly.) Similarly, in some instances, the original codon at a particular position may already be the target codon (i.e., $X_j^0 = X_j^0(h)$), in which case no replacement occurs. Accordingly, it should be understood that, in a given application, $\varphi_{co} \le \pi$.

Those skilled in the art with the benefit of this disclosure will appreciate that process 200 can improve the likelihood that codon replacement will result in a desired effect on reproductive fitness. For example, in the G protein of human respiratory syncytial virus A (RSVA), the most preferred codon encoding threonine at locus 80 is ACG. However, across the entire genome, ACG is least preferred. A conventional genome-based codon de-optimization method would replace other codons at locus 80 with ACG. However, because ACG is most preferred at locus 80, the conventional method may have the effect of optimizing rather than de-optimizing reproductive fitness of the organism. In contrast, process 200 can result in selecting a codon other than ACG for locus 80 of the G protein of RSVA, increasing the likelihood that de-optimization is achieved. Such effects may be more consequential for codon optimization, where accidental de-optimization of a few codons may defeat the optimization purpose.

### *k*-mer Segment-Based Codon Replacement

Process 200 operates on codons, which correspond to 3 consecutive bases in a nucleotide sequence. In some embodiments, process 200 can be modified to perform *k*-mer segment-based codon replacement (kSCR), where a *k*-mer is a group of *k* consecutive monomers in a nucleotide sequence. The value of *k* can be chosen as desired (provided that $k \geq 1$). For any given value of *k*, there are $4^k$ distinct *k*-mers. For example, if *k* = 2, the possible dinucleotides for DNA are {AA, AT, AC, AG, TA, TT, TC, TG, CA, CT, CC, CG, GA, GT, GC, GG}. If *k* = 3, each (non-overlapping) k-mer can be a codon. If *k* = 6, each k-mer can be a pair of adjacent codons.

In the kSCR approach, *k*-mers are considered synonymous if one k-mer can be replaced by another without altering the corresponding amino acid sequence. For example, consider the nucleotide sequence UUCGAU, which codes for the amino acid sequence "FD" (per FIG. 1A). Considering *k*-mers of length *k* = 3, the same amino acid sequence can be synonymously coded to UUC**GAC** (replacing GAU with GAC) or **UUU**GAU (replacing UUC with UUU). Considering *k*-mers of length *k* = 2, the amino acid sequence UUCGAU can be synonymously coded to UUCG**AC** (replacing AU with AC), U**UU**GAU (replacing UC with UU), or UU**UG**AU (replacing CG with UG). As the frequency of dinucleotides at a particular position may be different from the frequency of codons, the recoded result may be different between *k* = 2 and *k* = 3. Accordingly, the recoded sequence can depend on the length of the *k*-mer chosen to calculate frequencies (or probability scores). For a given segment of s nucleotides in a genetic sequence, a synonymous recoding using *k*-mers of length *k* < s can change, at most, (*s* - *k* + 1) *k*-mers. For codon optimization, *k*-mers can be replaced by more-frequently-occurring synonymous *k*-mers, while for codon de-optimization, *k*-mers can be replaced by less-frequently-occurring synonymous *k*-mers.

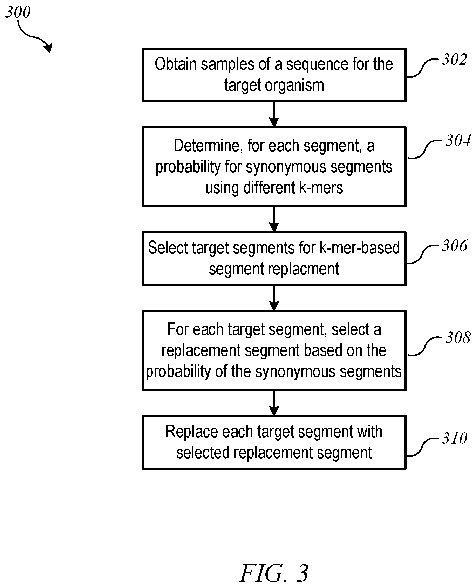

FIG. 3 shows a flow diagram of a process 300 for modifying a genome according to some embodiments. Process 300 can be performed for a variety of organisms, including pathogens such as viruses. Process 300 is similar to process 200, except that substitution is performed for *k*-mers of arbitrary length *k*.

At block 302, samples of a genetic sequence for the target organism are obtained. As in process 200, genetic sequences for an organism can be obtained using conventional techniques for extracting and sequencing DNA or RNA, and the genetic sequence can include a portion or all of the genome of the target organism. Samples can be extracted from individual organisms and sequenced. For some organisms (e.g., various strains of influenza virus), genetic databases are available and can be used. It is assumed that a number *N* of samples are obtained. As before, samples are distinguished by a sample index *i*, where $1 \leq i \leq N$, and the sequence has a length of *J* amino acids (or *J* codons). In process 300, the sequence is divided into a number (*B*) of non-overlapping segments of length *k*, and a segment index *j* can be defined such that $1 \leq j \leq B$.

At block 304, for each segment *j*, a probability score (e.g., frequency) can be determined for each *k*-mer. In some embodiments, the *k*-mer at segment *j* of a target sequence can be denoted as $Y_j^0$, and the *k*-mer observed at segment *j* in the ith sample can be denoted as $Y_j^i$. A set of observed *k*-mers for segment *j* can be defined as {$W_j(r)$}, where index *r* identifies a particular *k*-mer at segment *j*, and $R_j$ denotes the number of *k*-mers for a particular segment ($1 \leq r \leq R_j$). In general, not all $4^k$ possible *k*-mers are synonymous for a given segment, and $1 \leq R_j \leq 4^k$. A segment-specific probability score for a particular *k*-mer (index *r*) at a particular segment *j* ($1 \leq j \leq B$) can be computed as:

$$p_j(r) = \sum_{i=1}^{N} I\big(W_j(r) = Y_j^i\big) / N \,. \qquad (8)$$

A global probability score for a target segment can also be computed. For example, $Y_j(a)$ can denote a segment of s nucleotides that is synonymous to $Y_j^0$, where index *a* distinguishes different segments of length *s*. A global frequency $P_j(a)$ of a particular synonymous segment $Y_j(a)$ can be computed according to

$$P_j(a) = \sum_{r=1}^{R_j} I\big(W_j(r) = Y_j(a)\big) \cdot p_j(r) \,. \qquad (9)$$

That is, $P_j(a)$ is the sum of observed *k*-mers in the segment, weighted by the frequency observed for each *k*-mer. Similarly, a global frequency for the target segment $Y_j^0$ can be computed according to

$$P_j = \sum_{r=1}^{R_j} I\left(W_j(r) = Y_j^0\right) \cdot p_j(r). \qquad (10)$$

In this manner, a $k$-mer bias profile can be established for the organism.

At block 306, a set of target segments to be modified can be selected. The set of target segments can be represented as $\psi$, and the number of target segments can be represented as $|\psi|$. In some embodiments, every segment can be selected as a target segment, in which case $|\psi| = B$. In other embodiments, the target segments can be a proper subset of the total number of segments, in which case $|\psi| < B$. Selection of target segments can be random, or the selection can be based on prior biological information and/or statistical information, similarly to process 200.

At block 308, for each target segment, a replacement segment is selected. For example, a replacement segment $Y_j(a)$ can be selected from the set of available segments $\{Y_j(r), 1 \le r \le R_j\}$. In some embodiments, the replacement segment can be selected based on the probability scores and a desired effect of replacement. For instance, for codon optimization, the index $H$ of the most preferred synonymous segment $Y_j(a)$ can be determined according to:

$$H = \arg\max\{a | P_j(a)\}. \qquad (11)$$

Similarly, for codon de-optimization the index $L$ of the least preferred synonymous segment $Y_j(a)$ can be determined according to:

$$L = \arg\min\{a | P_j(a)\}. \qquad (12)$$

It should be noted that indexes $H$ and $L$ are segment-specific. As with process 200, selection of a replacement segment for each segment can be made independently, e.g., based on the probability scores of different segments at a given location within the genome, and different replacement segments can be selected for the same original segment at different locations within the genome. Selecting the most-preferred segment can result in codon optimization, while selecting the least-preferred segment can result in codon de-optimization.

At block 310, for at least one instance of the organism, replacement of segments can be performed. In particular, at each target segment, the existing segment can be replaced by the replacement $k$-mer selected for that segment at block 308. Thus, if $Y_j(b)$ denotes the segment selected at block 308, then for each target location $j \in \psi$, the replacement

$Y_j^0 \leftarrow Y_j^0(b)$ is performed. For codon optimization, $b = H$ can be used, and for codon de-optimization, $b = L$ can be used. As in process 200, replacement of segments can be performed using existing techniques, such as designing appropriate primers for PCR (polymerase chain reaction) or other amplification reactions. In addition or instead, any specific polynucleotide sequence (such as a modified sequence determined at block 208) can be chemically synthesized, especially if it is of a relatively shorter length.

It should be understood that in the case where $k = 3$ and $B = J$, process 300 can be the same as process 200 ($Y_j = X_j$).

In the case where $k = 2$, kSCR process 300 can capture CpG and UpA combinations, which are known to affect gene expression. Replacement at such sites can be performed according to objectives of optimization or de-optimization. For instance, a replacement that induces incrementing of the CG content is likely to result in reduced virus replication due to hyper-methylation.

**Interaction-Based Selection of Codons for Replacement**

In processes 200 and 300, selection of locations (or segments) where replacement occurs and selection of the replacement codon or $k$-mer can be made independently for each location (or segment). In some embodiments, interaction-based effects can be taken into account when selecting locations (or segments) for replacement and/or the replacement codon or $k$-mer. For example, genetic interaction is known to play a vital role in the evolution of a pathogen and in maintaining overall fitness. Mutations may appear in a concerted manner. For instance, it is often observed that the effective mutations underlying seasonal influenza epidemics appear in groups. Accordingly, sabotaging genetic interactions may help to reduce overall fitness of a virus or other pathogen.

For example, two (or more) positions within the genome that exhibit statistical correlations, which suggest genetic interactions, can be targeted together for replacement with synonymous codons (or other *k*-mers). A variety of metrics can be used to identify statistical correlations. One example is linkage disequilibrium (LD), which evaluates non-randomness of a relationship between two loci.

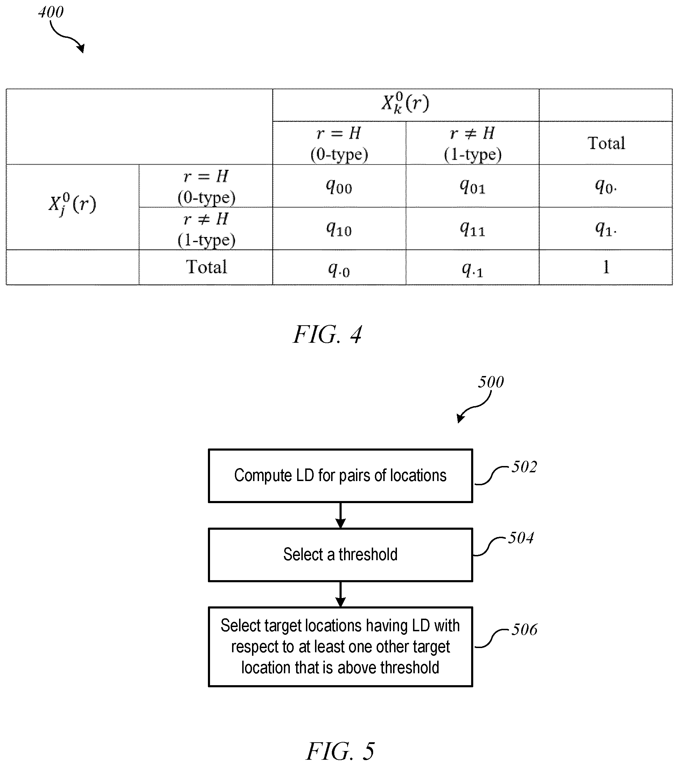

Linkage disequilibrium between two loci can be computed using a contingency table. FIG. 4 shows an example of a contingency table 400 for two positions (*j* and *k*) in a genetic sequence. $X_j^0(r)$ denotes a codon at position *j*, and $X_k^0(r)$ denotes a codon at position *k*. Any two positions $1 \leq j, k \leq J, j \neq k$) can be considered. Probability $q_{0\cdot}$ indicates the probability that codon $X_j^0(r)$ is the most-preferred codon (*r = H*) for location *j*, and probability $q_{1\cdot}$ indicates the probability that codon $X_j^0(r)$ is not the most-preferred codon (*r ≠ H*) for location *j*.

Similarly, probability $q_{\cdot 0}$ indicates the probability that codon $X_k^0(r)$ is the most-preferred codon (*r = H*) for location *k*, and probability $q_{\cdot 1}$ indicates the probability that codon $X_k^0(r)$ is not the most-preferred codon (*r ≠ H*) for location *k*. Joint probabilities are indicated as $q_{00}$ (both codons are most preferred at their respective locations), $q_{11}$ (neither codon is most preferred at its location); $q_{01}$ (codon $X_j^0(r)$ is the most-preferred codon for location *j* and codon $X_k^0(r)$ is not the most-preferred codon for location *k*); and $q_{10}$ (codon $X_k^0(r)$ is the most-preferred codon for location *k* and codon $X_j^0(r)$ is not the most-preferred codon for location *j*). In some embodiments, linkage disequilibrium LD can be computed as:

$$LD_{jk} = r_{jk}^2 = \frac{D_{jk}^2}{q_{\cdot 0} \cdot q_{\cdot 1} \cdot q_{0\cdot} \cdot q_{1\cdot}}, \tag{13}$$

where

$$D_{jk} = q_{00} - (q_{\cdot 0} \cdot q_{0\cdot}) = (q_{00} \cdot q_{11}) - (q_{01} \cdot q_{10}). \tag{14}$$

Other methods for computing LD can also be used.

In some embodiments, LD can be employed to select some or all of the target locations to be modified in a process such as process 200. FIG. 5 shows a flow diagram of a process 500 for selecting target locations according to some embodiments. Process 500 can be used, e.g., at block 206 of process 200.

At block 502, linkage disequilibrium $LD_{jk}$ can be computed (e.g., according to Eq. (13)) for a number of different pairs of locations (*j, k*). In some embodiments, a comprehensive approach can be used where $LD_{jk}$ is computed for every pair of locations (*j, k*) satisfying $1 \leq j, k \leq J, j \neq k$.

At block 504, a threshold (*d*) for a statistically significant LD can be selected. The threshold can depend on how LD is defined; for Eq. (13), $0 < d \leq 1$. In some embodiments, the threshold *d* can be selected based on considerations related to the nature of the genome of the target organism. For instance, in the genome of SARS-CoV-2, *d* = 0.1 can be selected; for respiratory syncytial virus (RSV), *d* = 0.2 can be selected.

At block 506, a set (*τ*) of target locations can be selected such that each target location in the set *τ* has LD above threshold *d* with respect to at least one other location. For example, the set of target locations can be defined as:

$$\tau = \{j | LD_{jk} \geq d, 1 \leq j, k \leq J, j \neq k\}, \tag{15}$$

where $LD_{jk}$ is given by Eq. (13).

In some embodiments, the set *τ* can be the set of target locations selected at block 206 of process 200. If desired, additional target locations can also be selected. Codon pair de-optimization (e.g., at blocks 208 and 210 of process 200) can be performed by replacing each codon of the pair with the least-preferred synonymous codon at that location. That is, for $j \in \tau$, the replacement $X_j^0 \leftarrow X_j^0(L)$ can be performed, where $X_j^0(L)$ is the least-preferred codon at location $j$, as described above. A proportion of de-optimization ($\varphi_{cpd}$) can be used to represent the proportion of synonymous replacement conducted using codon-pair selection based on LD.

In various embodiments, other measures of correlation between pairs codons can be used. Examples include chi-squared test, W-test, a co-mutation test, or any other quantity that reveals statistical correlations between pairs of codons at different positions. In the example described above, $LD_{jk}$ is computed for each codon pair ($j$, $k$) in a genetic sequence of the target organism. Other techniques can be used to identify correlations on different scales, e.g., within a gene segment, a whole-genome, a specific viral strain or species, or the like. Further, while use of LD is described in the context of codon de-optimization, similar techniques can be applied to codon optimization. (For instance, in the context of codon optimization, high LD may be an indication that replacement of a codon at a particular location is not desirable.) In some embodiments, LD-based selection of replacement locations can be applied to $k$-mers of any desired length $k$.

**Position-Based Codon Optimization Toward Multiple Hosts**

In some embodiments, a position-based codon process such as process 200 can be used to modulate codon usage of a pathogen (e.g., a virus) in one host species ("host 1") toward the usage in a different host species ("host 2"). For instance, in a vaccine manufacturing process, host 1 can be the species the vaccine is to be applied to (e.g., human beings) while host 2 is the organism used for culturing and replicating the virus (e.g., an insect expression system). Such modulation can be accomplished by selecting a replacement codon that is more preferred, though not necessarily most preferred, in both species. For example, the set of preferred codons $\omega_j$ for amino acid $A_j^0$ in host 1 can be defined as:

$$\omega_j = \left\{ X_j^0(r) \middle| p_j(r) \geq c \right\}, \tag{16}$$

where $0 < c \leq 0.5$.

Position-based codon usage data for a given virus in host 2 may be unavailable due to sample limitations. Accordingly, genomic coding usage in the genome of host 2 can be considered. The frequency of amino acid $A_j^0$ of the target sequence in the genome of host 2 can be denoted as $q_j^0$, and the frequency of alternative codons for amino acid $A_j^0$ in the genome of host 2 can be denoted as $q_j^0(r)$, where $1 \leq r \leq 6$. For some host organisms, codon usage data is available in public databases.

The set of synonymous codons more preferred than $X_j^0$ for amino acid $A_j^0$ in host 2 can be defined as

$$\theta_j = \left\{ X_j^0(r) \middle| q_j^0(r) > q_j^0 \right\}. \tag{17}$$

If $\omega_j \cap \theta_j \neq \varnothing$, then the preferred codons for amino acid $A_j^0$ in both hosts can be defined as

$$X_j^0(e) \in \omega_j \cap \theta_j. \tag{18}$$

Replacement can be performed in the manner described above. For example, a proportion of planned replacement ($0 < \pi \leq 1$) can be selected, and a subset of codon positions can be chosen as the target locations $\delta$ such that $\pi = |\delta|/J$. In some embodiments, some or all of the target locations $\delta$ can be loci with high genome interactions (e.g., elements of set $\tau$ as defined above). At each location $j \in \delta$, the replacement $X_j^0 \leftarrow X_j^0(e)$ is performed. In other words, at each target location $j \in \delta$, the original codon is replaced with a codon that is preferred in both hosts. A proportion

of optimization ($\varphi_{coh}$) can be used to represent the proportion of synonymous replacement conducted for $j \in \delta$ using the replacement $X_j^0 \leftarrow X_j^0(e)$.

The invention is summarized as follows:

1. A method of modifying a genome, the method comprising:

obtaining a plurality of samples of a genetic sequence of a target organism;
determining, for each of a plurality of target locations in the genetic sequence, a location-specific probability score for each of a plurality of synonymous codons; and
for each target location:

selecting, based on the location-specific probability scores for the target location, a replacement codon; and
replacing, in a genomic molecule, an existing codon at the target location with the replacement codon.

2. The method of item 1, wherein determining the probability score for a particular synonymous codon includes determining a fraction of the samples of the genetic sequence that include the particular synonymous codon at the target segment.

3. The method of item 1, wherein the replacement codon has a highest probability score among the synonymous codons at the target segment.

4. The method of item 1, wherein the replacement codon has a lowest probability score among the synonymous codons at the target segment.

5. The method of item 1 further comprising:

computing, for each of a plurality of pairs of locations in the genetic sequence, a linkage disequilibrium parameter; and
selecting at least some of the target locations based on the linkage disequilibrium parameter.

6. The method of item 5, wherein the target locations are selected such that each target location has a linkage disequilibrium with respect to at least one other target location that is above a threshold.

7. The method of item 1, wherein the target locations include every location for which two or more synonymous codons exist.

8. The method of item 1, wherein the target organism is a pathogen.

9. The method of item 1, wherein the target organism is a virus and the location-specific probability scores are determined based on samples of the virus genetic sequence obtained from host organisms belonging to a first species.

10. The method of item 9 further comprising:

determining a global probability score for each of a plurality of synonymous codons based on samples of the virus genetic sequence obtained from host organisms belonging to a second species,
wherein the replacement codon is selected based in part on the location-specific probability scores and based in part on the global probability scores.

11. The method of item 10 further comprising:

computing, for each of a plurality of pairs of locations in the genetic sequence, a linkage disequilibrium parameter; and
selecting at least some of the target locations based on the linkage disequilibrium parameter.

12. A method of modifying a genome, the method comprising:

obtaining a plurality of samples of a genetic sequence of a target organism;
determining, for each of a plurality of target segments in the genetic sequence, a probability score for each of a set of synonymous segments, wherein a synonymous segment is a segment obtained by replacing a k-mer in the target segment with a different $k$-mer without affecting a corresponding amino acid sequence, wherein each target segment has a length $s$ and $s \geq k$; and
for each target segment:

selecting, based on the probability scores for the target segment, a replacement segment from the set of synonymous segments; and

replacing, in a genomic molecule, the target segment with the replacement segment.

13. The method of item 12, wherein determining the probability score for a synonymous segment includes determining a sum of available *k*-mers in the segment, weighted by the *k*-mer frequencies observed in the samples.

14. The method of item 12, wherein the replacement segment has a highest probability score among the synonymous segments at the target segment.

15. The method of item 12, wherein the replacement segment has a lowest probability score among the synonymous segments at the target segment.

16. The method of item 12, wherein *k* = 3 and each *k*-mer corresponds to a codon.

17. The method of item 12, wherein *k* = 2 and each *k*-mer corresponds to a dinucleotide.

18. The method of item 12, wherein *k* = 6.

19. The method of item 18, wherein each *k*-mer corresponds to a pair of adjacent codons.

20. The method of item 12 further comprising:

computing, for each of a plurality of pairs of segments in the genetic sequence, a linkage disequilibrium parameter; and

selecting at least some of the target segments based on the linkage disequilibrium parameter.

21. The method of item 12, wherein the target segments are selected such that each target segment has a linkage disequilibrium with respect to at least one other target segment that is above a threshold.

22. The method of item 12, wherein the target segments include every segment for which two or more synonymous segments exist.

23. The method of item 12, wherein the target organism is a pathogen.

**Examples**

A target genetic sequence, specifically the Hemagglutinin of A/Michigan/45/2015(H1N1) influenza strain, was used to compute codon usage and evaluate de-optimization efficacy. A total of 19,747 sequences of the hemagglutinin of influenza virus from 2017 to 2019 were used to calculate codon usage. Five different codon de-optimization methods were applied, including: (1) an implementation of process 200 in which all codons are selected as target locations (referred to in this section as "Method A1"); (2) an implementation of process 200 with target locations selected according to process 500 (referred to in this section as "Method B"); (3) a conventional genome-based codon de-optimization technique ("Genome-based CD"); (4) a conventional genome-based codon pair de-optimization technique ("Genome-based CPD"); and (5) a conventional codon de-optimization technique that enhances CpG and UpA content.

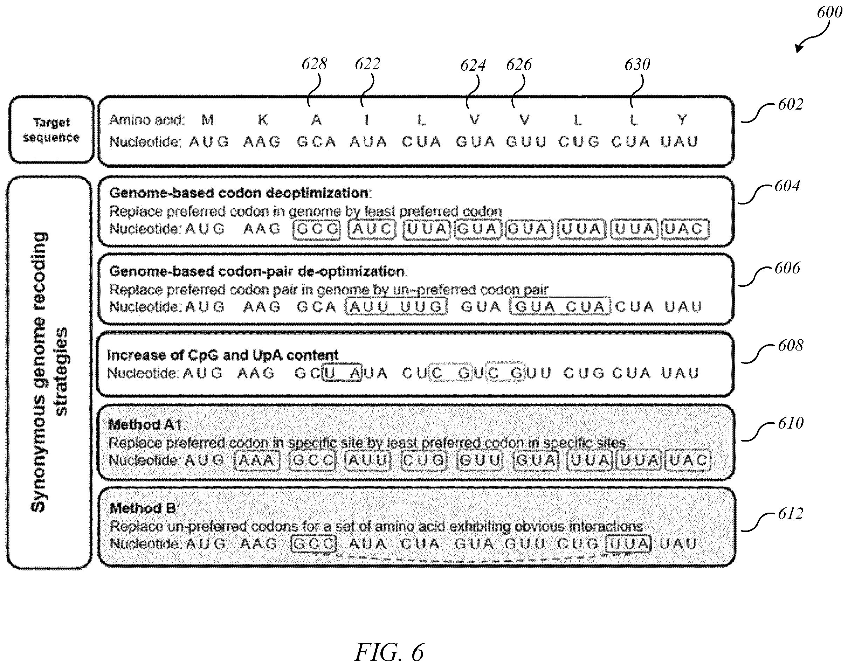

FIG. 6 shows a table 600 illustrating differences in codon replacements using different methods. At row 602, an initial sequence is shown, including the amino acids and the preferred codon for each amino acid. Rows 604, 606, and 608 show replacements made according to conventional methods: row 604 shows genome-based CD; row 606 shows genome-based CPD; and row 608 shows enhancement of CpG and UpA content. Replacements are circled as an aid to visualization. Rows 610 and 612 show replacements made using Method A1 and Method B. Genome-based CD (row 604) results in prevailing use of a particular codon for a given amino acid, such as UCG for serine (S) and UUA for lysine (L). Genome-based CPD (row 606) preserves the frequency of codons but shuffles synonymous codons to change the codon-pair bias. CpG and UpA enhancement increases the frequency of the CG and UA dinucleotides without changing the amino acid sequence.

As shown in FIG. 6, Method A1 (row 610) results in different substitutions from conventional genome-based CD. For example, in the target sequence (row 602), the fourth position 622 has codon AUA, which codes for isoleucene (I). Method A1 replaces codon AUA with codon AUU, which is the least-preferred codon at the fourth position 622, while conventional genome-based codon de-optimization (row 604) replaces codon AUA with codon AUC, which is the least-preferred codon across the genome. As another example, sixth position 624 and seventh position 626 each have codons that code for valine (V). Conventional genome-based codon de-optimization (row 604) replaces both codons with GUA (which is least-preferred across the genome). In contrast, Method A1 replaces the codon at sixth position 624 with GUU and the codon at seventh position 626 with GUA, based on which codon is least preferred at each position.

As further shown in FIG. 6, Method B (row 612) identifies non-adjacent codons with significant interactions (e.g., the codons at the third position 628 and the ninth position 630) and replaces each codon with the least-preferred codon at that position. In contrast, genome-based CPD (row 606) considers only adjacent codons.

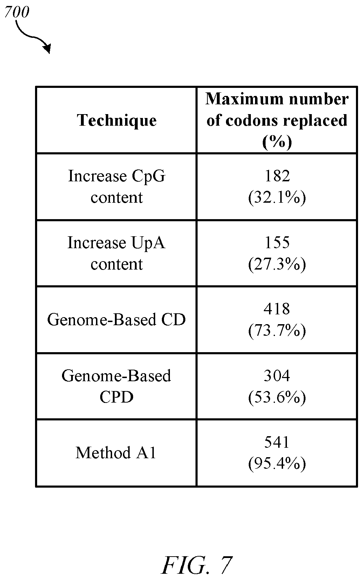

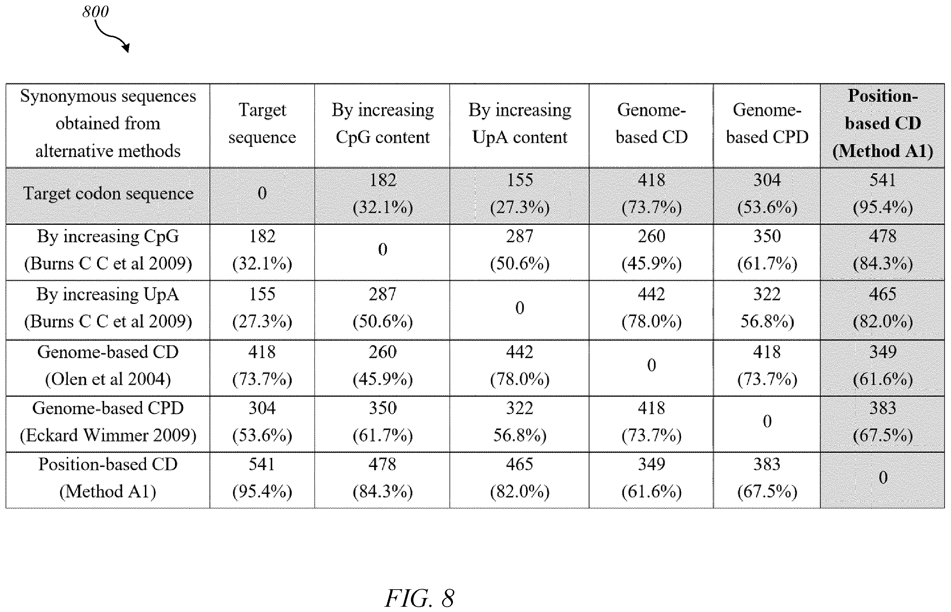

Additional demonstration of the differences between Method A1 and conventional codon de-optimization techniques is shown in FIGs. 7 and 8. There are a total of 567 codons in the Hemagglutinin of influenza A/H1N1. FIG. 7 is a table 700 showing, for each of five different techniques, the maximum number (and percentage) of the 567 codons that can be replaced using that technique. As shown, Method A1 can replace up to 541 codons ($\varphi_A$ = 95.4%), the largest among the techniques considered. The upper limit for Genome-Based CD is much lower, at 73.7%, and other techniques have even lower proportion of de-optimization.

FIG. 8 is a table 800 illustrating Hamming distance between sequences generated from the target sequence (Hemagglutinin of influenza A/H1N1) according to different strategies at their respective maximum recoding settings. For table 800, the Hamming distance between two sequences is defined as the number of codons that are different between the two sequences. The Hamming distance is shown in table 800 as a number and as a percentage of 567 total bases. The last column of table 800 shows that more than half of the codons in the sequence resulting from Method A1 are different from the codons in the target sequence or in any of the other modified sequences. This shows that Method A1 can produce de-optimized sequences with features that are distinct from conventionally-generated de-optimized sequences.

## Additional Embodiments

While the invention has been described with reference to specific embodiments, those skilled in the art will appreciate that variations and modifications are possible. A variety of techniques can be used to select target locations, and replacement codons at a particular location can be selected based on different criteria, including optimization or de-optimization of reproductive fitness.

Methods and systems of the kind described herein can be applied in a variety of contexts. For example, in some embodiments, location-specific probability scores for codons or other $k$-mers can be used to establish a position-dependent codon bias profile for a gene or genome. As described above, the codon bias profile can be used as a database for performing codon optimization or de-optimization. Profiling codon usage bias in the manner described herein may also facilitate a deeper understanding of the process of pathogen adaptation to a host and may provide insight into the evolutionary path of a pathogen, priority of mutation sites, mechanisms of pathogen-host interaction, and/or pathogen interaction with human or other animal genomes.

As another example, methods of the kind described herein can be used to generate de-optimized sequences for pathogens, e.g., as antigens in live-attenuated vaccines, with better safety and stability profiles as compared to conventional methods. A codon-de-optimized virus, for instance, can have a slower replication rate and a faster degeneration rate, resulting in a safer vaccine with fewer side effects. Further, a structurally and systematically de-optimized sequence as produced using techniques described herein would be genetically conserved, as compared to a sequence de-optimized at only a few codons, resulting in lower likelihood of vaccine-derived virus in the host. Specific examples of vaccines where methods of the kind described herein may be useful include vaccines targeting influenza viruses and RSV.

As yet another example, methods of the kind described herein can be used to generate optimized sequences for pathogens, thereby increasing the replicative fitness of the pathogen in a target organism (e.g., avian cell, insect cell, or the like). As one specific example, a codon-optimized recombinant protein may have improved replicative fitness in the baculovirus expression vector system and may deliver better yield of antigens for vaccine manufacture.

Certain aspects of the methods described herein can be implemented using software programs executing on computer systems of conventional design or other computer systems. For example, computation of probability scores for synonymous codons (or $k$-mers) at particular locations can be automated, as can selection of replacement codons. Other aspects of the methods described herein, e.g., modification of genetic molecules such as RNA or DNA, involve manipulation of chemical structures rather than data bits.

Computer programs incorporating features of the present invention that can be implemented using program code may be encoded and stored on various computer readable storage media; suitable media include magnetic disk or tape, optical storage media such as compact disk (CD) or DVD (digital versatile disk), flash memory, and other non-transitory media. (It is understood that "storage" of data is distinct from propagation of data using transitory media such as carrier waves.) Computer readable media encoded with the program code may include an internal storage medium of a compatible electronic device and/or external storage media readable by the electronic device that can execute the code. In some instances, program code can be supplied to the electronic device via Internet download or other transmission paths.

Accordingly, although the invention has been described with respect to specific embodiments, it will be appreciated that the invention is intended to cover all modifications and equivalents within the scope of the following claims.

**Claims**

1. A method of modifying a genome, the method comprising:

   obtaining a plurality of samples of a genetic sequence of a target organism;
   determining, for each of a plurality of target segments in the genetic sequence, a probability score for each of a set of synonymous segments, wherein a synonymous segment is a segment obtained by replacing a $k$-mer in the target segment with a different $k$-mer without affecting a corresponding amino acid sequence, wherein each target segment has a length $s$ and $s \geq k$; and
   for each target segment:

   selecting, based on the probability scores for the target segment, a replacement segment from the set of synonymous segments; and
   replacing, in a genomic molecule, the target segment with the replacement segment.

2. The method of claim 1, wherein determining the probability score for a synonymous segment includes determining a sum of observed k-mers in the segment, weighted by the $k$-mer frequencies observed in the samples.

3. The method of claim 1, wherein determining the probability score for a particular synonymous segment includes determining a location-specific probability score based on a fraction of samples having the particular synonymous segment at a particular location in the genetic sequence.

4. The method of claim 1, wherein the replacement segment has a highest probability score among the synonymous segments at the target segment.

5. The method of claim 1, wherein the replacement segment has a lowest probability score among the synonymous segments at the target segment.

6. The method of claim 1 further comprising:

   computing, for each of a plurality of pairs of segments in the genetic sequence, a linkage disequilibrium parameter; and
   selecting at least some of the target segments based on the linkage disequilibrium parameter.

7. The method of claim 1, wherein the target segments are selected such that each target segment has a linkage disequilibrium with respect to at least one other target segment that is above a threshold.

8. The method of claim 1, wherein the target segments include every segment for which two or more synonymous segments exist.

9. The method of claim 1, wherein the target organism is a pathogen.

10. The method of claim 1, wherein the target organism is a virus and the location-specific probability scores are determined based on samples of the virus genetic sequence obtained from host organisms belonging to a first species..

11. The method of any one of claims 1 to 10, wherein $k$ = 3 and each $k$-mer corresponds to a codon.

12. The method of claim 11, wherein $s$ = $k$ and each segment also corresponds to a codon.

13. The method of any one of claims 1 to 10, wherein $k$ = 2 and each $k$-mer corresponds to a dinucleotide.

14. The method of any one of claims 1 to 10, wherein $k$ = 6.

15. The method of claim 14, wherein each $k$-mer corresponds to a pair of adjacent codons.

| 1st base | 2nd base | | | | | | | | 3rd base |
|---|---|---|---|---|---|---|---|---|---|
| | U | | C | | A | | G | | |
| U | UUU | Phenylalanine (F) | UCU | Serine (S) | UAU | Tyrosine (Y) | UGU | Cysteine (C) | U |
| | UUC | | UCC | | UAC | | UGC | | C |
| | UUA | Leucine (L) | UCA | | UAA | Stop codon | UGA | Stop codon | A |
| | UUG | | UCG | | UAG | Stop codon | UGG | Tryptophan (W) | G |
| C | CUU | | CCU | Proline (P) | CAU | Histidine (H) | CGU | Arginine (R) | U |
| | CUC | | CCC | | CAC | | CGC | | C |
| | CUA | | CCA | | CAA | Glutamine (Q) | CGA | | A |
| | CUG | | CCG | | CAG | | CGG | | G |
| A | AUU | Isoleucine (I) | ACU | Threonine (T) | AAU | Asparagine (N) | AGU | Serine (S) | U |
| | AUC | | ACC | | AAC | | AGC | | C |
| | AUA | | ACA | | AAA | Lysine (K) | AGA | Arginine (R) | A |
| | AUG | Methionine (M) | ACG | | AAG | | AGG | | G |
| G | GUU | Valine (V) | GCU | Alanine (A) | GAU | Aspartic acid (D) | GGU | Glycine (G) | U |
| | GUC | | GCC | | GAC | | GGC | | C |
| | GUA | | GCA | | GAA | Glutamic acid (E) | GGA | | A |
| | GUG | | GCG | | GAG | | GGG | | G |

## FIG. 1A

| 1st base | 2nd base | | | | | | | | 3rd base |
|---|---|---|---|---|---|---|---|---|---|
| | T | | C | | A | | G | | |
| T | TTT | Phenylalanine (F) | TCT | Serine (S) | TAT | Tyrosine (Y) | TGT | Cysteine (C) | T |
| | TTC | | TCC | | TAC | | TGC | | C |
| | TTA | Leucine (L) | TCA | | TAA | Stop codon | TGA | Stop codon | A |
| | TTG | | TCG | | TAG | Stop codon | TGG | Tryptophan (W) | G |
| C | CTT | | CCT | Proline (P) | CAT | Histidine (H) | CGT | Arginine (R) | T |
| | CTC | | CCC | | CAC | | CGC | | C |
| | CTA | | CCA | | CAA | Glutamine (Q) | CGA | | A |
| | CTG | | CCG | | CAG | | CGG | | G |
| A | ATT | Isoleucine (I) | ACT | Threonine (T) | AAT | Asparagine (N) | AGT | Serine (S) | T |
| | ATC | | ACC | | AAC | | AGC | | C |
| | ATA | | ACA | | AAA | Lysine (K) | AGA | Arginine (R) | A |
| | ATG | Methionine (M) | ACG | | AAG | | AGG | | G |
| G | GTT | Valine (V) | GCT | Alanine (A) | GAT | Aspartic acid (D) | GGT | Glycine (G) | T |
| | GTC | | GCC | | GAC | | GGC | | C |
| | GTA | | GCA | | GAA | Glutamic acid (E) | GGA | | A |
| | GTG | | GCG | | GAG | | GGG | | G |

## FIG. 1B

*200*

Obtain samples of a sequence for the target organism ⟋*202*

↓

Determine, for each location, a probability for each synonymous codon ⟋*204*

↓

Select target locations for codon replacment ⟋*206*

↓

At each target location, select a replacement codon based on the probability of the synonymous codons at the target location ⟋*208*

↓

Replace codon at each target location with selected replacement codon ⟋*210*

*FIG. 2*

*300*

Obtain samples of a sequence for the target organism — *302*

↓

Determine, for each segment, a probability for synonymous segments using different k-mers — *304*

↓

Select target segments for k-mer-based segment replacment — *306*

↓

For each target segment, select a replacement segment based on the probability of the synonymous segments — *308*

↓

Replace each target segment with selected replacement segment — *310*

*FIG. 3*

400

| | | $X_k^0(r)$ | | Total |
|---|---|---|---|---|
| | | $r = H$ (0-type) | $r \neq H$ (1-type) | Total |
| $X_j^0(r)$ | $r = H$ (0-type) | $q_{00}$ | $q_{01}$ | $q_{0\cdot}$ |
| | $r \neq H$ (1-type) | $q_{10}$ | $q_{11}$ | $q_{1\cdot}$ |
| | Total | $q_{\cdot 0}$ | $q_{\cdot 1}$ | 1 |

*FIG. 4*

500

| Compute LD for pairs of locations | 502 |

↓

| Select a threshold | 504 |

↓

| Select target locations having LD with respect to at least one other target location that is above threshold | 506 |

*FIG. 5*

*FIG. 6*

| Technique | Maximum number of codons replaced (%) |
|---|---|
| Increase CpG content | 182 (32.1%) |
| Increase UpA content | 155 (27.3%) |
| Genome-Based CD | 418 (73.7%) |
| Genome-Based CPD | 304 (53.6%) |
| Method A1 | 541 (95.4%) |

700

*FIG. 7*

| Synonymous sequences obtained from alternative methods | Target sequence | By increasing CpG content | By increasing UpA content | Genome-based CD | Genome-based CPD | Position-based CD (Method A1) |
|---|---|---|---|---|---|---|
| Target codon sequence | 0 | 182 (32.1%) | 155 (27.3%) | 418 (73.7%) | 304 (53.6%) | 541 (95.4%) |
| By increasing CpG (Burns C C et al 2009) | 182 (32.1%) | 0 | 287 (50.6%) | 260 (45.9%) | 350 (61.7%) | 478 (84.3%) |
| By increasing UpA (Burns C C et al 2009) | 155 (27.3%) | 287 (50.6%) | 0 | 442 (78.0%) | 322 56.8%) | 465 (82.0%) |
| Genome-based CD (Olen et al 2004) | 418 (73.7%) | 260 (45.9%) | 442 (78.0%) | 0 | 418 (73.7%) | 349 (61.6%) |
| Genome-based CPD (Eckard Wimmer 2009) | 304 (53.6%) | 350 (61.7%) | 322 56.8%) | 418 (73.7%) | 0 | 383 (67.5%) |
| Position-based CD (Method A1) | 541 (95.4%) | 478 (84.3%) | 465 (82.0%) | 349 (61.6%) | 383 (67.5%) | 0 |

*FIG. 8*

EP 4 187 544 A1

**EP 4 187 544 A1**

Europäisches
Patentamt
European
Patent Office
Office européen
des brevets

# EUROPEAN SEARCH REPORT

Application Number

EP 22 20 9795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/024749 A1 (CONSEJO SUPERIOR DE INVESTIG CIENTÍFICAS [ES]; UNIV SALAMANCA [ES]) 8 February 2018 (2018-02-08) * see p. 4, 6, 31, fig. 1,5,7,9 * ----- | 1-4,6-13 | INV. G16B25/00 G16B20/00 G16B40/20 G16B20/40 |
| X | WO 2017/218727 A1 (HARVARD COLLEGE [US]) 21 December 2017 (2017-12-21) * p.16- 17, 64, fig. 5A,6, 36, 37, 39 * ----- | 1,14,15 | ADD. C12Q1/68 |
| X | US 2017/067030 A1 (WIMMER ECKARD [US] ET AL) 9 March 2017 (2017-03-09) * 24, 43, 103, 112 and fig. 1 * ----- | 1,5,9,10 | |
| A,P | GUPTA MANOJ KUMAR ET AL: "Next-generation development and application of codon model in evolution", FRONTIERS IN GENETICS, vol. 14, 1 January 2023 (2023-01-01), XP093030493, Switzerland ISSN: 1664-8021, DOI: 10.3389/fgene.2023.1091575 * the whole document * ----- | 1-15 | |

TECHNICAL FIELDS
SEARCHED       (IPC)

G16B
C12Q

The present search report has been drawn up for all claims

| Place of search | Date of completion of the search | Examiner |
|---|---|---|
| Munich | 10 March 2023 | Lüdemann, Susanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................
& : member of the same patent family, corresponding document

EPO FORM 1503 03.82 (P04C01)

23

**EP 4 187 544 A1**

## ANNEX TO THE EUROPEAN SEARCH REPORT
## ON EUROPEAN PATENT APPLICATION NO.

EP 22 20 9795

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2023

| Patent document cited in search report | Publication date | Patent family member(s) | Publication date |
|---|---|---|---|
| WO 2018024749 A1 | 08-02-2018 | NONE | |
| WO 2017218727 A1 | 21-12-2017 | CA 3027882 A1 | 21-12-2017 |
| | | CN 109997192 A | 09-07-2019 |
| | | EP 3472319 A1 | 24-04-2019 |
| | | JP 7062861 B2 | 09-05-2022 |
| | | JP 2019519233 A | 11-07-2019 |
| | | JP 2022046554 A | 23-03-2022 |
| | | US 2020055903 A1 | 20-02-2020 |
| | | US 2022246240 A1 | 04-08-2022 |
| | | WO 2017218727 A1 | 21-12-2017 |
| US 2017067030 A1 | 09-03-2017 | AU 2008232491 A1 | 09-10-2008 |
| | | BR PI0809600 A2 | 12-07-2016 |
| | | CA 2682089 A1 | 09-10-2008 |
| | | CN 103476425 A | 25-12-2013 |
| | | DK 2139515 T3 | 27-08-2018 |
| | | EP 2139515 A2 | 06-01-2010 |
| | | EP 3431099 A1 | 23-01-2019 |
| | | HR P20181319 T1 | 19-10-2018 |
| | | JP 5733976 B2 | 10-06-2015 |
| | | JP 2010523086 A | 15-07-2010 |
| | | JP 2015091247 A | 14-05-2015 |
| | | KR 20100019423 A | 18-02-2010 |
| | | MX 349825 B | 15-08-2017 |
| | | PL 2139515 T3 | 30-11-2018 |
| | | US 2010209454 A1 | 19-08-2010 |
| | | US 2017067030 A1 | 09-03-2017 |
| | | US 2019010469 A1 | 10-01-2019 |
| | | US 2022298492 A1 | 22-09-2022 |
| | | WO 2008121992 A2 | 09-10-2008 |

EPO FORM P0459

For more details about this annex : see Official Journal of the European Patent Office, No. 12/82

24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63283910